(19) Europäisches Patentamt
European Patent Office
Office européen des brevets

(11) Veröffentlichungsnummer: **0 005 793**
**A1**

# (12) EUROPÄISCHE PATENTANMELDUNG

(21) Anmeldenummer: **79101581.1**

(22) Anmeldetag: **22.05.79**

(51) Int. Cl.[2]: **C 07 C 121/64**
C 07 C 120/00, C 09 K 3/34

(30) Priorität: **23.05.78 DE 2822504**

(43) Veröffentlichungstag der Anmeldung:
**12.12.79 Patentblatt 79/25**

(84) Benannte Vertragsstaaten:
**CH FR GB IT NL**

(71) Anmelder: **SIEGFRIED AKTIENGESELLSCHAFT**
**CH-4800 Zofingen(CH)**

(72) Erfinder: **Granwehr, Bernhard, Dr.**
**Döbeligut 3**
**CH-4800 Zofingen(CH)**

(72) Erfinder: **Gnehm, René, Dr.**
**Bornweg 8**
**CH-4665 Küngoldingen(CH)**

(74) Vertreter: **Jaeger, Klaus, Dr. rer. nat. et al,**
**JAEGER, GRAMS & PONTANI Patentanwälte**
**Bergstrasse 48 1/2**
**D-8035 München-Gauting(DE)**

## (54) Verfahren zur Herstellung von trans-4-Alkyl-cyanoarylcyclohexanen.

(57) Ein Verfahren zur Herstellung von trans-4-Alkyl-cyanoaryl-cyclohexanen der allgemeinen Formel

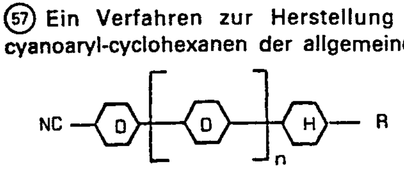

(I),

in der R ein unverzweigter oder einmal verzweigter aliphatischer Kohlenwasserstoffrest mit bis zu 12 Kohlenstoffatomen ist und n den Wert Null, 1 oder 2 hat.

Ausgehend von einer entsprechenden Arylcarbonsäure der allgemeinen Formel

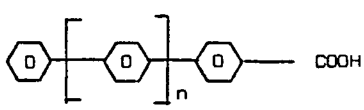

(II)

wird diese zunächst einer Birch-Reduktion und anschließend nach veresterung in Gegenwart von Pd/Aktivkohle zu einem Gemisch der beiden cis-trans-isomeren Cyclohexyl-4-carbonsäure-ethylester hydriert. Durch anschließendes Erhitzen des Estergemisches unter absolut wasserfreien Bedingungen in Ethanol in Gegenwart von Natrium und unter Inertgas wird der im Isomerengemisch vorhandene cis-Ester quantitativ in den entsprechenden trans-Ester umgelagert. Der erhaltene stereochemisch reine trans-Ester wird dann stereospezifisch verseift. Im letzten Verfahrensschritt wird die Cyanogruppe eingeführt.

Die erhaltenen Verbindungen der allgemeinen Formel I eignen sich insbesondere als Komponenten von Flüssigkristallgemischen.

EP 0 005 793 A1

0005793



---

TITEL:

Verfahren zur Herstellung von trans-4-Alkyl-cyanoarylcyclohexanen

---

Die Erfindung betrifft ein Verfahren zur stereoselektiven Herstellung von trans-4-Alkyl-cyanoarylcyclohexanen der allgemeinen Formel

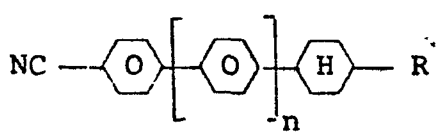

(I ),

in der R ein unverzweigter oder einmal verzweigter aliphatischer Kohlenwasserstoffrest mit 1 bis 12 Kohlenstoffatomen ist und n den Wert Null, 1 oder 2 hat.

Die trans-4-Alkyl-cyanoarylcyclohexane der allgemeinen Formel I sind in der Lage, nematische Flüssigkristallphasen zu bilden. Sie werden vorzugsweise als Komponenten von Flüssigkristallgemischen, insbesondere nematischen Flüssigkristallgemischen, verwendet.

Die trans-4-Alkyl-(4'-cyanophenyl)cyclohexane und die trans-4-Alkyl-(4"-cyanobiphenyl-4'-yl)cyclohexane sind bekannte Substanzen.

Die trans-4-Alkyl-(4"'-cyanoterphenyl-4'-yl)cyclohexane sind neue Substanzen. Als Komponenten von Flüssigkristallgemischen sind sie in der Lage, den Existenzbereich der Mesophase zu verbreitern und deren Stabilität zu verbessern, ohne die Schaltzeiten dieser Gemische wesentlich zu erhöhen.

Die 4-Alkyl-cyanoarylcyclohexane treten in einer cis-Form und in einer trans-Form auf. Für die Verwendung als Flüssigkristall oder Bestandteil eines Flüssigkristallgemisches ist nur die trans-Form brauchbar.

Ein Verfahren zur Herstellung von trans-4-Alkyl-(4'-cyanophenyl)cyclohexanen ist aus Angew. Chem. 89, 103 bekannt. 4-Alkylcyclohexanon wird mit Phenylmagnesiumbromid in Diethyläther zu einem Gemisch der beiden cis-trans-isomeren 4-Alkyl-1-phenyl-cyclohexanole umgesetzt. Das Isomerengemisch wird säulenchromatographisch getrennt. Das trans-Isomere wird mit Raney-Nickel, das cis-Isomere mit Palladium/Aktivkohle hydriert. Bei der Hydrierung des cis-Isomeren tritt Isomeriserung zum trans-Isomeren ein. Die beiden trans-4-Alkylphenylcyclohexanfraktionen werden vereinigt, nach Friedel-Crafts acetyliert, dem Haloformabbau zur Carbonsäure unterworfen, in das Säureamid überführt und schließlich mit $POCl_3$ zum Nitril dehydratisiert.

Nachteilig an diesem bekannten Verfahren ist, daß die 4-Alkylcyclohexanone schwer zugängliche Substanzen sind, daß eine säulenchromatographische Trennung der nur schwer trennbaren cis-trans-isomeren Cyclohexanole erforderlich ist und daß die chromatographisch getrennten Isomeren dann in getrennten Ansätzen hydriert werden müssen.

Angesichts dieses Standes der Technik liegt der Erfindung die Aufgabe zugrunde, ein Verfahren zur stereoselektiven Herstellung der trans-4-Alkyl-cyanoarylcyclohexane der eingangs genannten Art zu schaffen, das einfach und wirtschaftlich durchführbar ist, insbesondere von leicht zugänglichen und preiswerten Ausgangssubstanzen ausgeht und keine Isomerentrennung erfordert.

Gelöst wird diese Aufgabe durch ein Verfahren der eingangs genannten Art, das erfindungsgemäß die im kennzeichnenden Teil des Patentanspruchs 1 genannten Merkmale aufweist.

Die für das Verfahren benötigten Ausgangssubstanzen, die Biphenyl-, Terphenyl- oder Quaterphenyl-4-carbonsäuren, sind entweder im Handel erhältlich oder im Falle der Biphenyl-4-carbonsäure durch eine Grignard-Umsetzung aus dem entsprechenden 4-Bromderivat oder auch in den anderen Fällen z.B. nach H. Gilman (J.Org.Chem.22,446) durch Umsetzen mit Organolithium herstellbar.

Die durch die Reduktion als Isomerengemisch erhaltenen Cyclohexan-4-carbonsäureester können, ohne daß eine Trennung der cis-trans-Isomeren erforderlich ist, in wasserfreiem Ethanol unter Stickstoff in Gegenwart von Natrium, vorzugsweise in Gegenwart von ca. 10 Mol-% Natrium, quantitativ zum stereochemisch reinen trans-Arylcyclohexan-4-carbonsäureethylester isomerisiert werden. Wenn dabei das Methylestergemisch statt

des Ethylestergemisches der Isomerisierung unterzogen wird, findet eine Umesterung statt, so daß aufgrund der Isomerisierung in absolutem Ethanol in dieser Verfahrensstufe stets der trans-Ethylester erhalten wird.

Der so erhaltene trans-Arylcyclohexan-4-carbonsäureethylester ist in an sich bekannter Weise glatt und problemlos verseifbar. Die Verseifung erfolgt vorzugsweise mit Lithiumhydroxid-monohydrat in ethanolischer Lösung.

Die erhaltene trans-Arylcyclohexan-4-carbonsäure wird anschließend in üblicher Reaktion einer Kettenverlängerung und dann einer Reduktion der α-ständigen Carbonylgruppe unterzogen. Die Kettenverlängerung muß, wenn sie stereospezifisch verlaufen soll, bei tiefen Temperaturen, vorzugsweise bei - 50 bis - 100 °C erfolgen. Die Reaktion wird absolut wasserfrei, vorzugsweise in Tetrahydrofuran und/oder Hexan, durchgeführt. Wenn der aromatische Rest der Phenylrest ist, kann vor der Kettenverlängerung, vorzugsweise bereits vor der Verseifung, der Phenylrest perhalogeniert, vorzugsweise perchloriert, werden. Die Dehalogenierung kann dann vor oder nach der Reduktion der Carbonylgruppe erfolgen.

Bei der Auswahl der zahllosen möglichen Verfahren zur Kettenverlängerung ist darauf zu achten, daß eine Deprotonierung des Cyclohexans in 4-Stellung mit Sicherheit ausgeschlossen ist. Bereits die wegen der guten Ausbeuten vorzugsweise eingesetzte Methode der Kettenverlängerung durch Reaktion mit Alkyllithium verläuft dabei quantitativ stereospezifisch.

Die anschließende Reduktion der Carbonylgruppe kann in gebräuchlicher Weise, beispielsweise nach Clemmensen oder Wolff-Kishner erfolgen.

Das nach dieser Reduktion erhaltene trans-4-Alkyl-arylcyclohexan entspricht dem im bekannten Verfahren ebenfalls als Zwischenprodukt erhaltenen trans-4-Alkyl-phenylcyclohexan. Die Einführung der Cyanogruppe kann wie im bekannten Verfahren beschrieben über eine Acetylierung und einen Haloformabbau mit anschließender Dehydratisierung des Amids erfolgen. Vorzugsweise wird jedoch der überraschenderweise ebenfalls quantitativ stereospezifisch und mit hoher Ausbeute verlaufende Weg der direkten Umsetzung des trans-4-Alkyl-arylcyclohexans in Gegenwart von Aluminiumchlorid mit Oxalylchlorid nach Friedel-Crafts gewählt. Das erhaltene 4'-, 4"- bzw. 4"'- Säurechlorid kann dann praktisch in einer Stufe über das Amid in das Nitril überführt werden.

Statt der im Arylrest p-ständigen Cyanogruppe können selbstverständlich im Bedarfsfall auch andere Gruppen eingeführt werden, insbesondere Estergruppen. Entsprechend kann auch in 4-Stellung des Cyclohexans statt einer Kettenverlängerung und anschließenden Reduktion der Carbonylgruppe eine andere Modifizierung vorgenommen werden, beispielsweise eine Veresterung oder eine Umesterung mit Alkoholen, wie sie dem Fachmann an dieser Stelle für die Herstellung von Substanzen für nematische Flüssigkristallphasen an sich geläufig und bekannt sind.

Die Erfindung ist im folgenden anhand eines Ausführungsbeispiels näher erläutert.

Beispiel:

16,2 g (0,082 mol) im Handel erhältliche Biphenyl-4-carbonsäure werden in 30 g absolut wasserfreiem Ethanol und 300 ml wasserfreiem Ammoniak bei - 70 °C unter Stickstoff im Verlauf von 60 min in kleinen Portionen mit insgesamt 6,0 g Natrium versetzt. Die erhaltene Suspension wird nach Abschluß der Reaktion mit 50 g festem Ammoniumchlorid und 70 ml kaltem Wasser versetzt. Anschließend wird der Ammoniak unter vermindertem Druck abgezogen. Nach Verdünnen mit weiteren 30 bis 50 ml Wasser wird mit 100 ml Diethyläther extrahiert. Anschließend wird mit konzentrierter Salzsäure angesäuert und noch weitere dreimal mit Äther extrahiert. Die vereinigten Ätherphasen werden über Magnesiumsulfat getrocknet und eingeengt. Dabei fallen 14,6 g weiße kristalline Substanz aus, die als ein Isomerengemisch von cis- und trans-Phenylcyclohexa-2,5-dien-4-carbonsäure identifiziert wird.

10 g (0,050 mol) des so erhaltenen Isomerengemisches werden in 5 bis 10 ml Methanol gelöst, mit einer katalytischen Menge von p-Toluolsulfonsäure und mit 50 ml Tetrachlorkohlenstoff versetzt. Das Gemisch wird 10 h unter Rückfluß auf Siedetemperatur erhitzt. Nach dem Abkühlen und vollständigen Trennen der beiden Phasen wird die Tetrachlorkohlenstoffphase abgetrennt, eingeengt und in wasserfreiem Ethanol aufgenommen. Anschließend wird mit Palladium/Aktivkohle hydriert. Nach Aufnahme der berechneten Menge Wasserstoff wird der Katalysator durch Filtrieren abgetrennt. Das Reaktionsgemisch wird mit einigen Millilitern Benzol versetzt, um auch letzte Spuren von Wasser azeotrop zu entfernen. Das so getrocknete Reaktionsgemisch wird anschließend unter Stickstoff mit 10 Mol-% Natrium versetzt und 48 h unter Rückfluß auf Siedetemperatur erhitzt. Anschließend wird abgekühlt, eingeengt, in Wasser aufgenommen,

neutralisiert und mit Diethyläther extrahiert. Die vereinigten Ätherextrakte werden getrocknet. Anschließend wird das Lösungsmittel unter vermindertem Druck abgezogen. Dabei werden 10,5 g praktisch reinen, weißen trans-Phenyl hexan-4-carbonsäureethylesters erhalten.

10,0 g (0,043 mol) des erhaltenen stereochemisch reinen Esters werden in Ethanol gelöst und mit 2,7 g (0,065 mol) Lithiumhydroxidmonohydrat versetzt. Nach Abschluß der Verseifung wird das Reaktionsgemisch mit ethanolischer Salzsäure neutralisiert, eingeengt, mit Benzol azeotrop von Wasser befreit und in Tetrahydrofuran suspendiert. Die Suspension wird auf - 70 °C abgekühlt und unter Stickstoff mit 0,043 mol n-Butyllithium in Hexan versetzt. Das Reaktionsgemisch wird bei dieser Temperatur 1 h gerührt. Anschließend wird das Reaktionsgemisch aus dem Kryostaten genommen und bei Raumtemperatur auf ein Eis/Wasser-Gemisch gegossen. Dann wird neutralisiert, eingeengt und in Dioxan aufgenommen. Das erhaltene Keton wird dann ohne vorherige Isolierung direkt bei Raumtemperatur in üblicher Weise nach Clemmensen reduziert. Nach dem Umkristallisieren werden 5,6 g weißes trans-4-n-Pentylphenylcyclohexan erhalten.

In 4'-Stellung des Phenylrestes der so erhaltenen Substanz kann dann in der aus Angew. Chem. 89, 103 bekannten Weise die Cyanogruppe eingeführt werden.

Alternativ wird das erhaltene trans-4-n-Pentyl-phenylcyclohexan in Gegenwart von wasserfreiem Aluminiumchlorid mit Oxalylchlorid zum entsprechenden 4'-Säurechlorid umgesetzt und anschließend über das Amid und durch Dehydratisierung des Amids mit $POCl_3$ schließlich in das trans-

0005793

4-n-Pentyl-(4'-cyanophenyl)cyclohexan überführt, das einen Schmelzpunkt von 30 bis 30,5 °C und einen Klärpunkt von 55 °C aufweist. Die erhaltene Substanz zeigt nematisches Verhalten, so daß ein Vorliegen der äquatorialen trans-Form angenommen wird.

## Patentansprüche

1. Verfahren zur Herstellung von trans-4-Alkyl-cyanoaryl-cyclohexanen der allgemeinen Formel

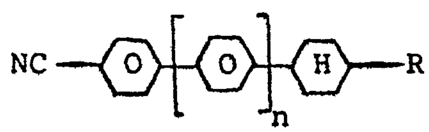

(I),

in der R ein unverzweigter oder einmal verzweigter aliphatischer Kohlenwasserstoffrest mit 1 bis 12 Kohlenstoffatomen ist und n den Wert Null, 1 oder 2 hat, dadurch gekennzeichnet, daß

(a) eine Arylcarbonsäure der allgemeinen Formel

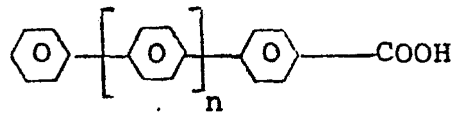

(II)

in der Kälte mit solvatisierten Elektronen in Gegenwart eines Protonendonators zu einem Gemisch der entsprechenden beiden cis-trans-isomeren Cyclohexa-2,5-dien-4-carbonsäuren

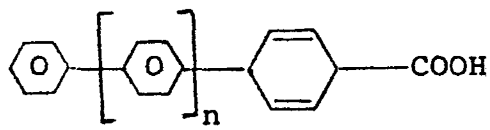

(III)

reduziert wird (Birch-Reduktion), daß

(b) die Carbonsäuren der allgemeinen Formel III mit Methanol oder Ethanol in Gegenwart katalytischer Mengen p-Toluolsulfonsäure verestert und anschließend in absolutem Ethanol unter praktisch vollständiger Umesterung der Methylester mit Pd/Aktivkohle zu den entsprechenden cis-trans-isomeren 1-arylsubstituierten Cyclohexan-4-carbonsäureethylestern

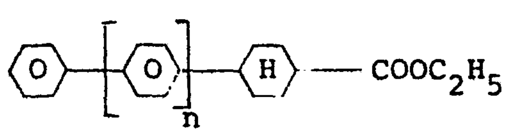

(IV)

hydriert werden, daß

(c) das Gemisch der beiden cis-trans-isomeren Cyclohexyl-4-carbonsäureethylester dann absolut wasserfrei unter Inertgas in Ethanol in Gegenwart von Natrium unter Rückfluß auf Siedetemperatur erhitzt wird, wobei der im Isomerengemisch vorhandene cis-Ester quantitativ zum entsprechenden trans-Ester isomerisiert wird, daß

(d) der dabei erhaltene reine 1-arylsubstituierte trans-Cyclohexan-4-carbonsäureethylester in an sich bekannter und gebräuchlicher Weise stereospezifisch verseift wird und die erhaltene freie trans-Cyclohexan-4-carbonsäure dann in bekannter Weise durch Kettenverlängerung bei tiefen Temperaturen mit anschließender Reduktion der Carbonylgruppe stereospezifisch in das entsprechende trans-Alkylderivat überführt wird und dass

(e) schließlich in 4'-Stellung, 4"Stellung bzw. 4'"-Stellung des aromatischen Substituenten die Cyanogruppe eingeführt wird.

2. Verfahren nach Anspruch 1, dadurch gekennzeichnet, daß die Reduktion der Stufe (a) bei ca. -70 °C mit einer Lösung von Natrium in wasserfreiem flüssigem Ammoniak in Gegenwart von wasserfreiem Ethanol als Protonendonator durchgeführt wird.

3. Verfahren nach einem der Ansprüche 1 oder 2, dadurch g e k e n n z e i c h n e t , daß der trans-Cyclohexan-4-carbonsäurethylester in der Stufe (d) in ethanolischer Lösung mit Lithiumhydroxidmonohydrat verseift wird und anschließend bei -70 °C absolut wasserfrei in Tetrahydrofuran mit Alkyllithium umgesetzt wird und das dabei erhaltene Alkyl-cyclohexylketon in gebräuchlicher Weise nach Clemmensen oder Wolff-Kishner reduziert wird.

4. Verfahren nach einem der Ansprüche 1 bis 3, dadurch g e k e n n z e i c h n e t , daß der aromatische Rest vor der Verseifung, zumindest vor der Isomerisierung, in der Stufe (d) perhalogeniert wird und nach der Isomerisierung wieder vollständig dehalogeniert wird.

5. Verfahren nach einem der Ansprüche 1 bis 4, dadurch g e k e n n z e i c h n e t , daß die Einführung der Cyanogruppe in der Stufe (e) durch Umsetzen des trans-4-Alkyl-arylcyclohexans nach Friedel-Crafts mit Oxalylchlorid und anschließendes Überführen des Säurechlorids in einer Stufe über das Säureamid in das Nitril erfolgt.

0005793

Nummer der Anmeldung

79101581.1

Europäisches Patentamt

# EUROPÄISCHER RECHERCHENBERICHT

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int.Cl.³) |
|---|---|---|---|
| D | Angew.Chemie, 89, Seite 10.. | 1 | C 07 C 121/64<br>C 07 C 120/00<br>C 09 K 3/34 |
| | DE-A-2 823 909 (F.Hoffmann-La Roche & Co AG)<br>* Seite1, Zeilen 1-13<br>Seite 4, Zeilen 8-11<br>Seite 6, Zeilen 9-13 * | 1 | |
| P | DD-A-131 094 (Merck Patent GmbH)<br>* Seite 3, Zeilen 15-18<br>Seite 6, Zeilen 8-22<br>Seite 7, Zeilen 1-16 * | 1 | |
| | DE-A-1 793 383 (Knoll AG, Chemische Fabriken)<br>* Seite 1, Zeilen 1-5 * | 1 | RECHERCHIERTE SACHGEBIETE (Int. Cl.³)<br>C 07 C 121/64<br>C 07 C 120/00<br>C 09 K 3/34 |
| | DE-A-2 639 838 (The Secretary of State)<br>* Seiten 1-16 * | 1 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 31.07.1979 | HEIN |